# EUROPEAN PATENT APPLICATION

(11) **EP 1 008 365 A1**
(43) Date of publication of application: **14.06.2000**
(21) Application number: 98901103.6
(22) Date of filing: 03.02.1998
(51) Int. Cl.: A61N 1/30

(54) **DEVICE FOR IONTOPHORESIS**

(30) Priority: 17.02.1997 JP 3209997
(71) Applicant: Kowa Co., Ltd., Naka-ku, Nagoya-shi, Aichi 460-0003 (JP)
(72) Inventor: KANEBAKO, Makoto, Fuji-shi Shizuoka 416-0939 (JP); INAGI, Toshio, Mishima-shi Shizuoka 411-0038 (JP); HAGA, Makoto, Shinjuku-ku Tokyo 162-0826 (JP); HAYASHI, Masahiro, Shinjuku-ku Tokyo 162-0826 (JP)
(74) Representative: Wächtershäuser, Günter, Prof. Dr.
(86) International application number: JP9800440
(87) International publication number: WO9835722

(57) **Abstract**

This invention relates to an iontophoretic device for the electrical percutaneous administration of a drug. As its features, a backing is provided with a power source, and a donor and a receptor are arranged on the same side of the backing with an insulating space region interposed therebetween, whereby the device has an integral structure. This iontophoretic device permits safe and efficient percutaneous absorption of a drug without increasing a current value and also features extremely good handling ease.

## Description

### Technical Field

This invention relates to a device for the electrical percutaneous administration of a drug, and more specifically to an iontophoretic device with a donor and a receptor arranged in an integral structure for the electrical percutaneous administration of a drug.

### Background Art

As methods for the administration of drugs, there are oral administration, transmucosal administration, transdermal administration, and the like. Transdermal administration features advantages such as 1) exhibition of pharmacological effects by a small amount of a drug, 2) possibility of topical administration, 3) avoidance of side effects on various organs, and 4) concerning peptide or protein preparations, avoidance of *in vivo* degradation. On the other hand, transdermal administration is accompanied by problems such as a) low absorption of a drug having a high molecular weight, b) low absorption of a hydrophilic drug, and c) difficult controllability of dose.

With a view to overcoming these problems, investigations have been made *inter alia* on methods making use of additives such as chemical enhancers, phonophoresis involving exposure to electromagnetic radiation such as ultrasonic waves, and iontophoresis featuring transport of a drug by an electric current.

Of these, the methods which make use of additives such as chemical enhancers have been studied with respect to aprotic polar solvents (Stoughton R.B., Fritsh W., Arch. Dermatol., No. 90, P. 512, 1964), surfactants (Woodford R., Barry B.W., J. Toxicol.-Cut & Ocular Toxicol., No. 5, P. 167, 1986), fatty acids and their derivatives (Cooper E.R., J. Pharm. Sci., No. 73, P. 1153, 1984), esters (Catz P., Friend D.R., J. Control Rel., No. 12, P. 171, 1990), etc. Although they are effective for hydrophobic drugs having relatively low molecular weights, they are accompanied by a problem such that stratum corneum may be separated or dissolved to cause a damage on skin, modification or degradation may be promoted for a peptide or protein preparation or dose control may be difficult. The use of phonophoresis is effective for the enhancement of percutaneous absorption (Bommanman D., Okuyama H., Stauffer P., Guy R., Pharm. Res., No. 9, P. 559, 1992), but this method is accompanied by a problem that, when exposed to electromagnetic radiation concurrently with administration, modification or degradation may be promoted or control of dose may be difficult. On the other hand, use of iontophoresis makes it possible to control the dose by suitably adjusting an electric current, although this method is accompanied by a problem that application of an excess current may promote a skin damage, such as burn, or degradation of a drug.

Concerning iontophoresis, percutaneous absorption enhancing effects of anionic drugs (Miller L.L., Smith G.A., Int. J. Pharm., No. 49, P. 15, 1989), cationic drugs (Siddiqui O., Roberts M.S., Polack A.E., J. Pharm. Pharmacol., No. 37, P. 732, 1985) and peptide or protein preparations (Banga A.K., Chien Y.W., Pharm. Res., No. 7, P. 10, S185, 1990) as well as percutaneous noninvasive extraction of nonionic substances based on the theorem of electroosmosis have been reported. Clinically, treatments of post herpetic neuralgia by using lidocaine, epinephrine, methylprednisolone or indomethacin (Ozawa Akira, NIHON IJI SHINPO, No. 3648, P. 25, 1994) have also been conducted.

In these treatments, however, a square or circular absorbent cotton impregnated with a drug solution and applied with an aluminum foil as an electrode is provided for use on a donor side, an absorbent cotton of a similar construction and impregnated with an aqueous solution of an electrolyte such as sodium nitrate is provided for use on a receptor side, they are brought into close contact with skin by surgical tapes or the like with an appropriate interval left therebetween, and a constant-current device called "Phoresor" is used. This method is accompanied by problems such that no effective absorption may be achieved due to low efficiency of electric quantity and insufficient contact between the electrodes and the absorbent cottons and between the absorbent cottons and the skin and the need for complex procedures and the large device makes it impossible to apply this method for the administration of a drug at home.

Although use of an increased current value is an only means for increasing an absorption rate or for achieving absorption of a low-absorption drug, such an increased current value has led to the problem of occurrence of burn as mentioned above. Hence, with a view to enhancing the absorption of a drug without increasing the current value, improvements have been made, for example, on i) voltage, ii) current application method, iii) electrode construction, iv) electrode pads, and the like as will be described next.
i) As a power source, a small battery has been disclosed, which is equipped with a relatively short service life and has a high internal impedance as producing no overcurrent even when a significant drop takes place in skin resistance due to sweating or the like (JP Kokai No. 3-254759).
ii) As a power application method, application of a pulsed current was studied to achieve depolarization of skin (Gupta, S.K., et al., J. Control Rel. No. 31, P. 229, 1994). This method is however accompanied by problems that it is effective only for certain specific drugs and that, as it requires a circuit and another power source for the production of a pulsed current, its apparatus is complex, has difficulty in operation and is costly.
iii) Disclosed as electrode constructions include plural donors and receptors arranged on the same flat surface with insulators interposed therebetween (JP Kokai No. 3-133464); plural donors and receptors concentrically arranged to control release of plural effective ingredients in accordance with a program (JP Kokai No. 4-208166); and a polygonal donor included within a receptor, said donor and said receptor being arranged on the same flat surface with an insulating part, which has a skin-contacting portion, interposed therebetween to avoid leakage of a current at the insulating part (JP Kokai No. 3-133464). Incidentally, although the plural receptors are used in JP Kokai No. 3-133464 to improve absorption, the absorption is not improved practically by increasing the number of receptors (see Test 9 to be described subsequently herein). Further, JP Kokai No. 4-208166, without any substantiation, states that a concentric arrangement of electrodes minimizes an electric current required for the release of a drug. However, even among concentric electrode constructions, the absorption varies depending on factors such as the width of the insulating part and the area ratio of the donors to the receptors (see Tests 10 and 11 to be described subsequently herein). In addition, it is an essential requirement in JP Kokai No. 8-185016 to have the skin-contacting portion in the insulating part. This skin-contacting portion has a stronger significance as an adhesion-assisting means for a drug-containing matrix having no adhesion to the skin, such as an agar gel, rather than inhibition of a deterioration in absorption as a result of leakage of a current, which flows between the donor and the receptor, on the skin surface.
iv) Disclosed as electrode pads include those having excellent peel strength to the skin even when water is contained therein and, by themselves, remaining free of changes in form and the like when a current is passed therethrough (JP Kokai No. 8-325545).

It is however the current situation that no satisfactory iontophoretic device is still unavailable despite these improvements.

An object of the present invention is therefore to provide an iontophoretic device which permits safe and efficient percutaneous absorption of a drug without increasing a current value and also has excellent handling ease.

### Disclosure of the Invention

The present invention have hence proceeded with a variety of research to achieve the above-described object, resulting in the completion of an iontophoretic device according to the present invention which is composed of a integral structure of a donor and a receptor.

Namely, the present invention has achieved the above-described object by an iontophoretic device for the electrical percutaneous administration of a drug, comprising: a backing provided with a power source, and a donor and a receptor arranged on the same one side of the backing with an insulating space region interposed therebetween, whereby the device has an integral structure.

### Brief Description of the Drawings

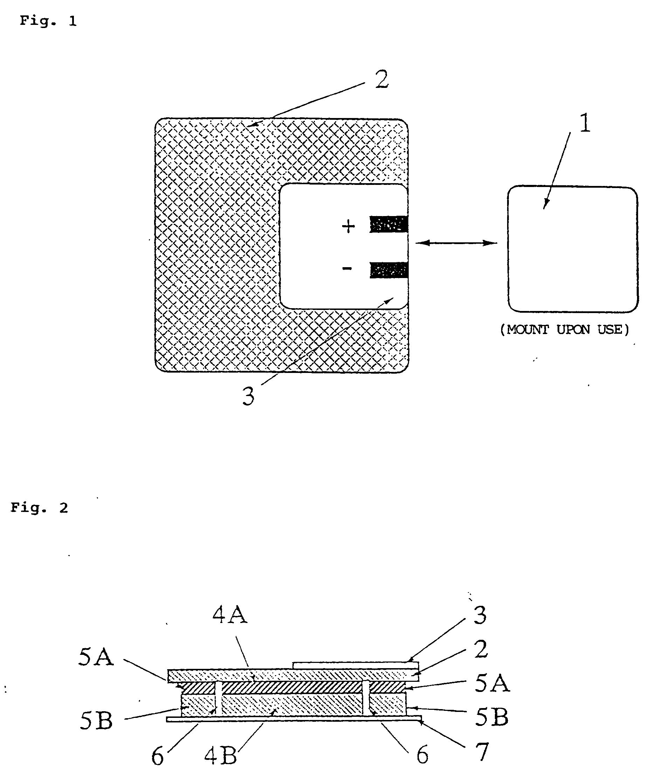

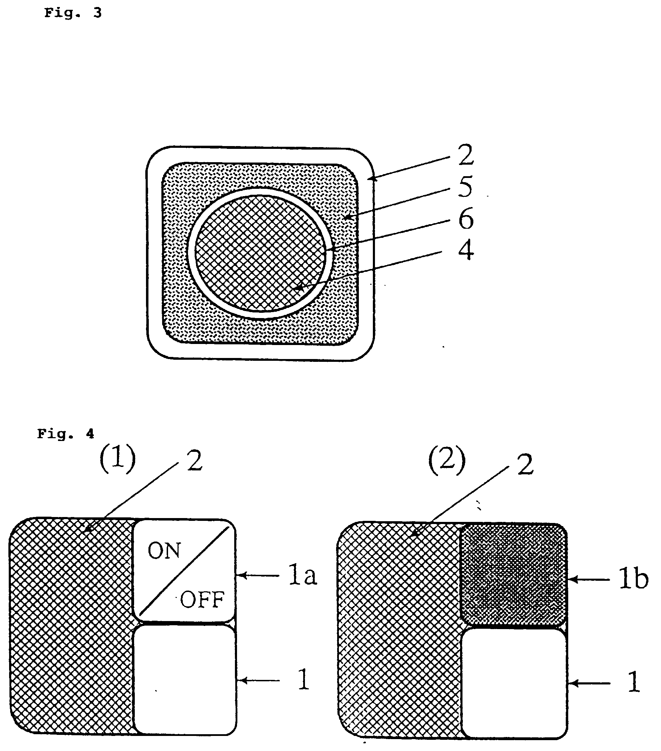

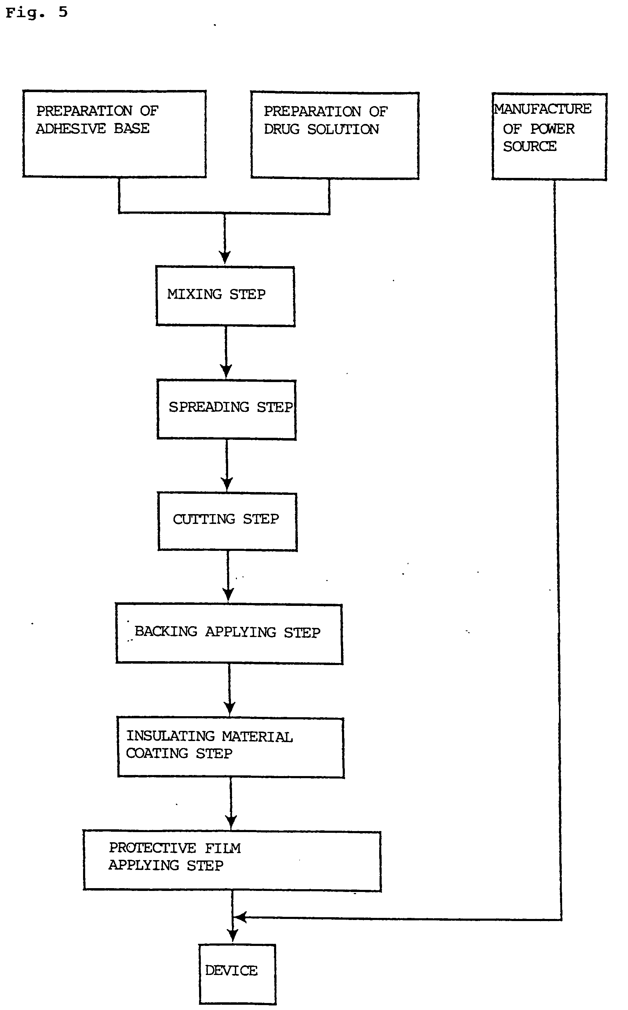

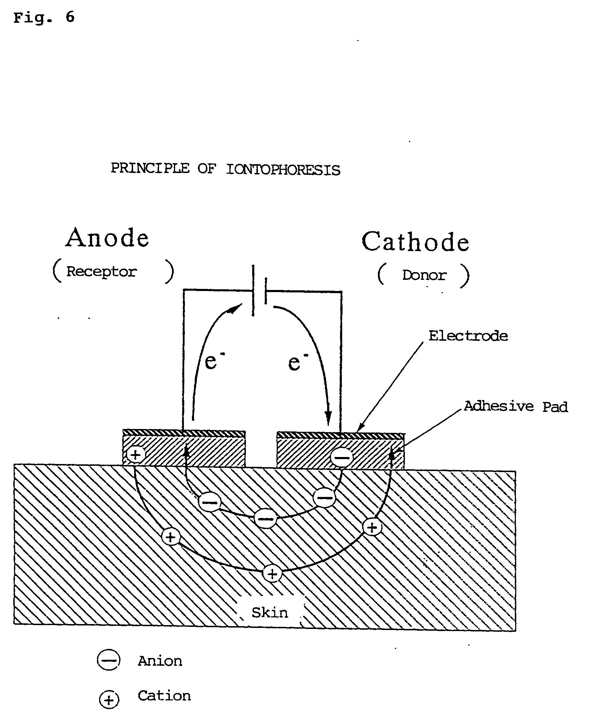

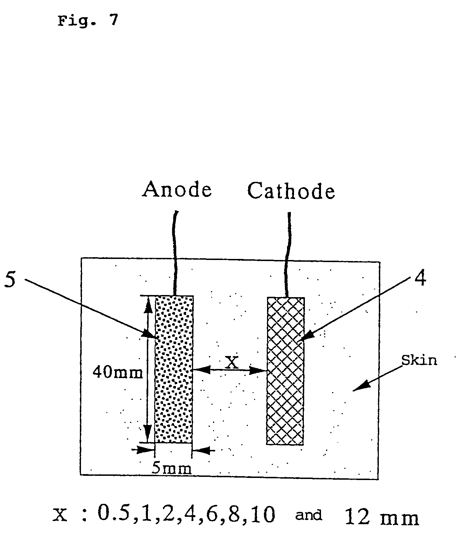

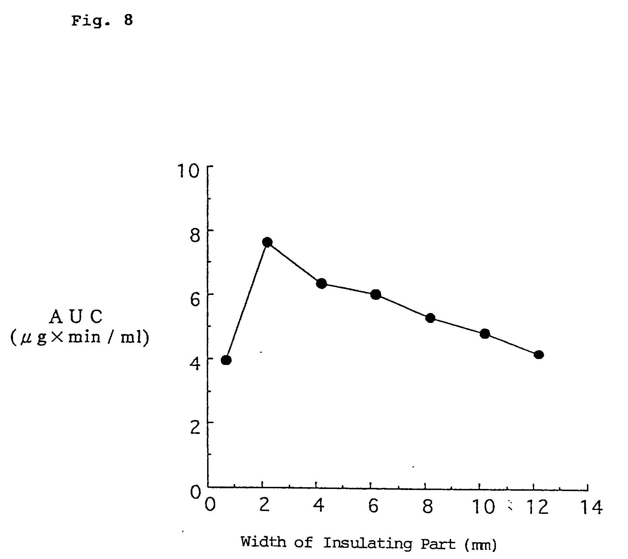

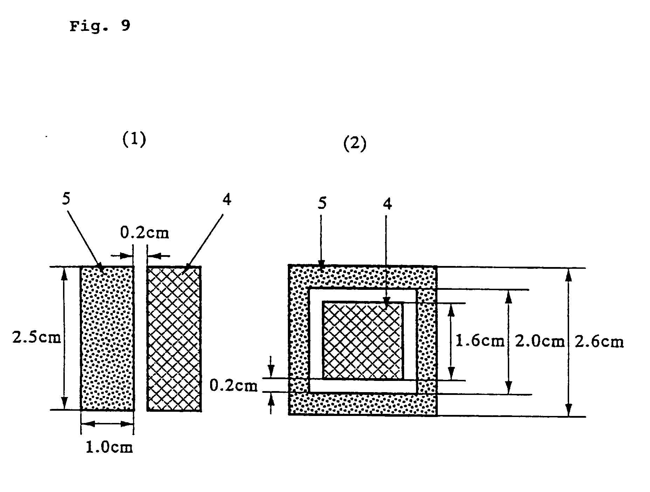

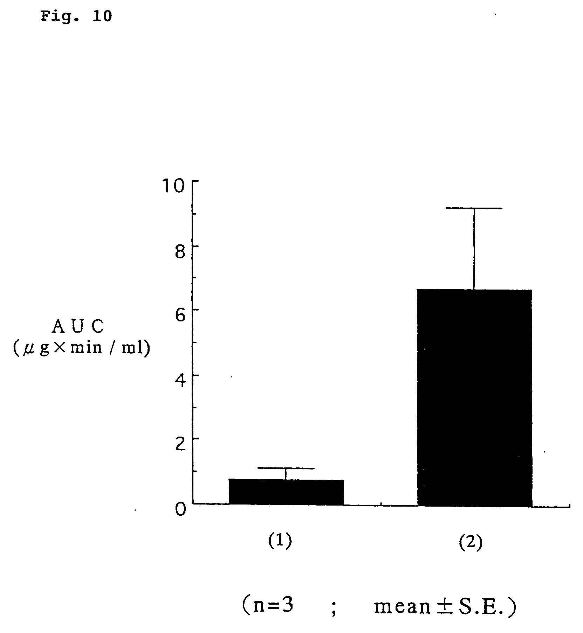

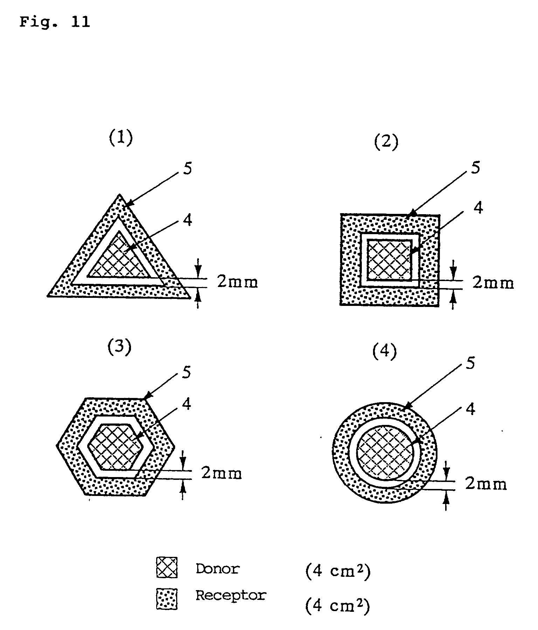

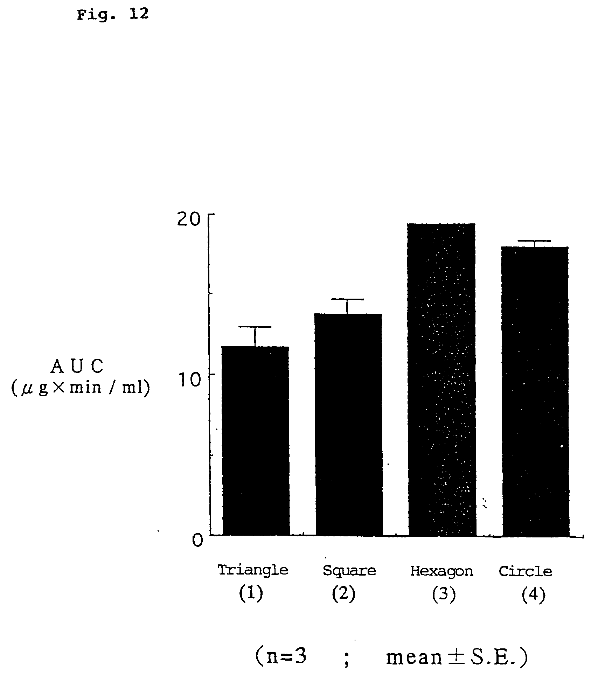

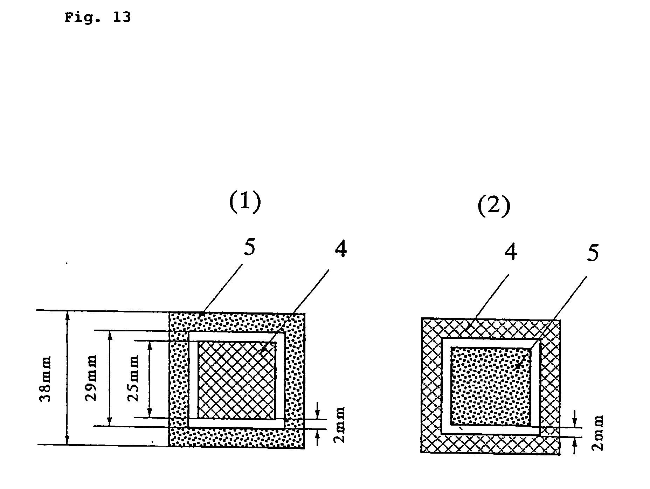

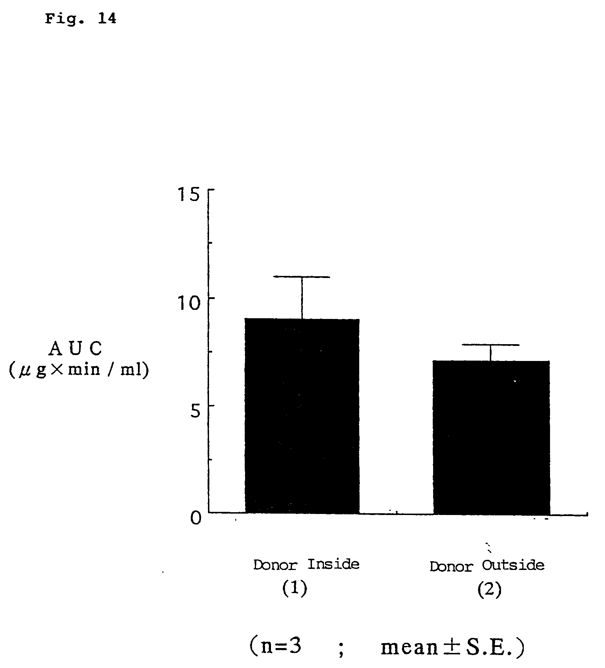

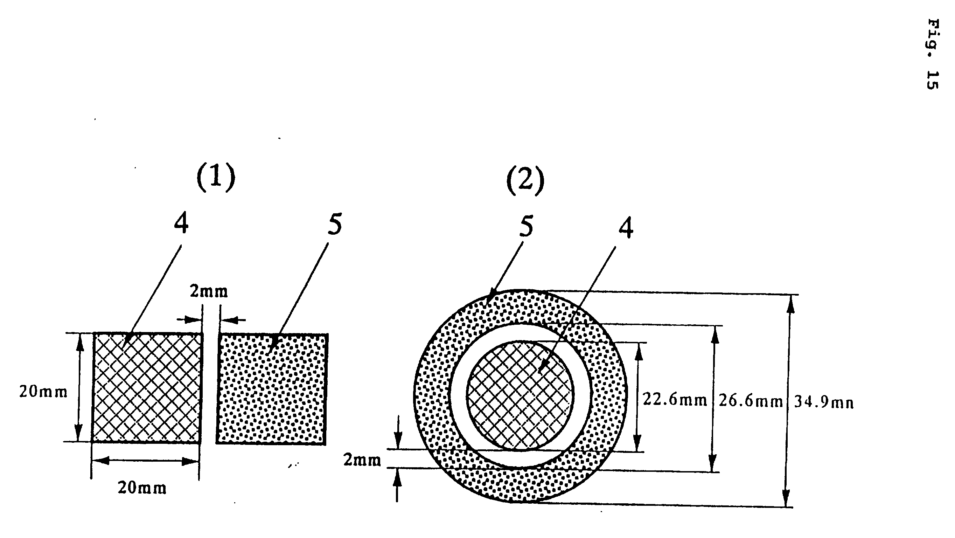

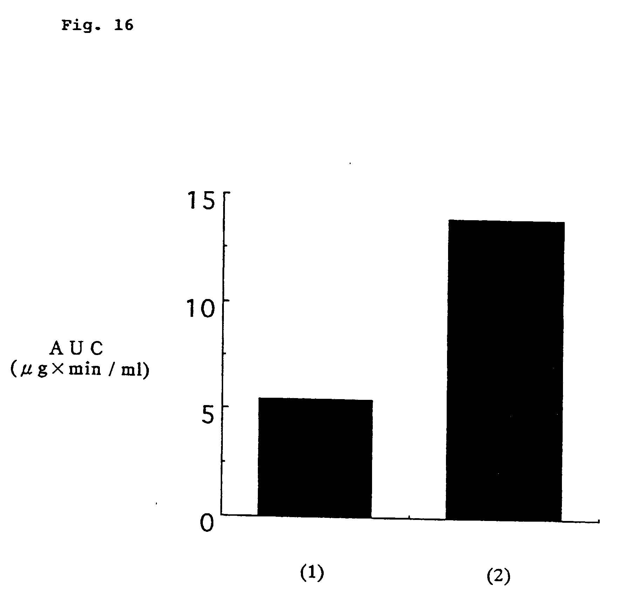

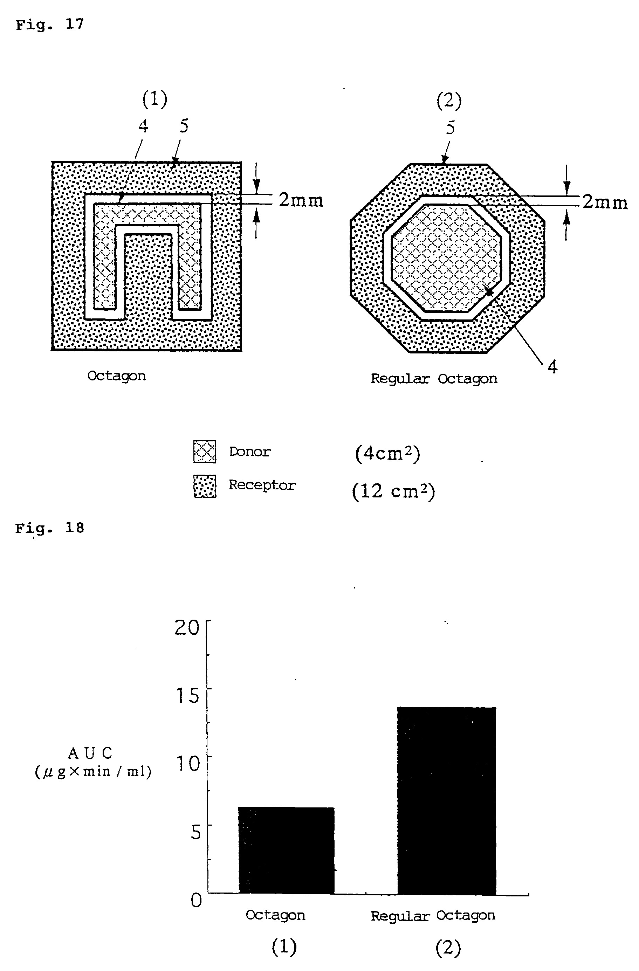

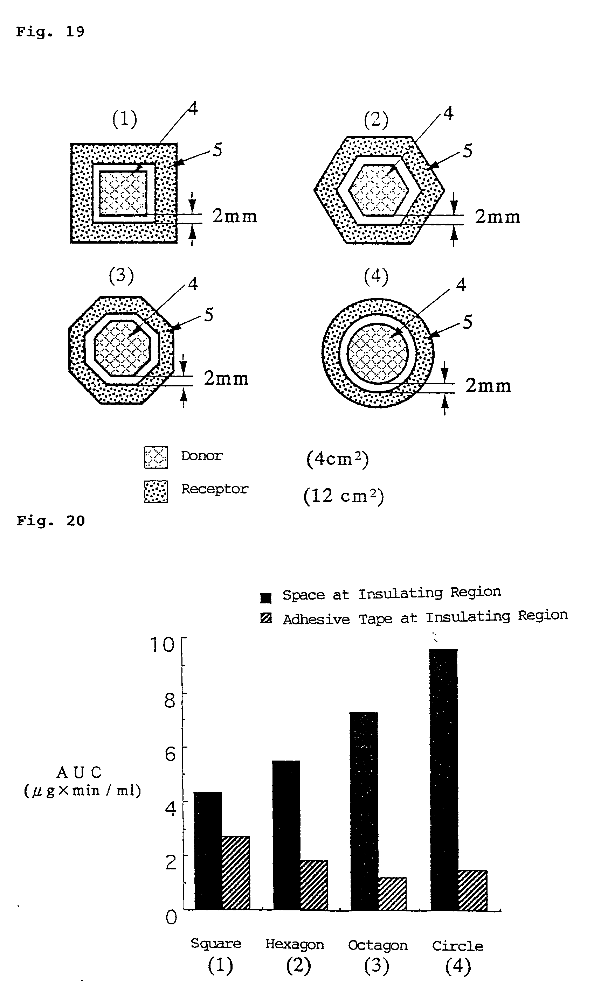

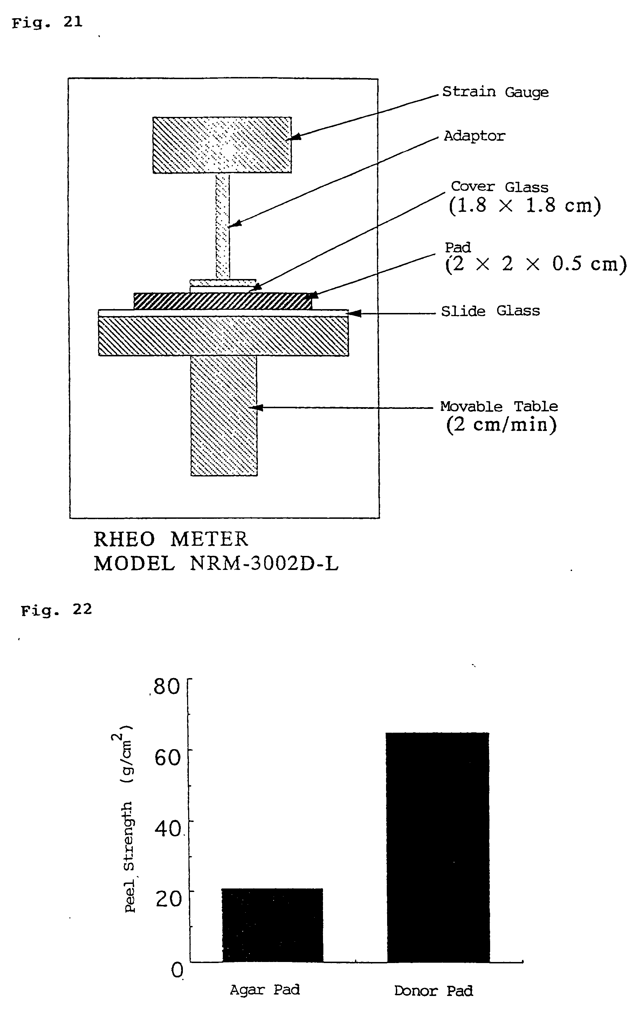

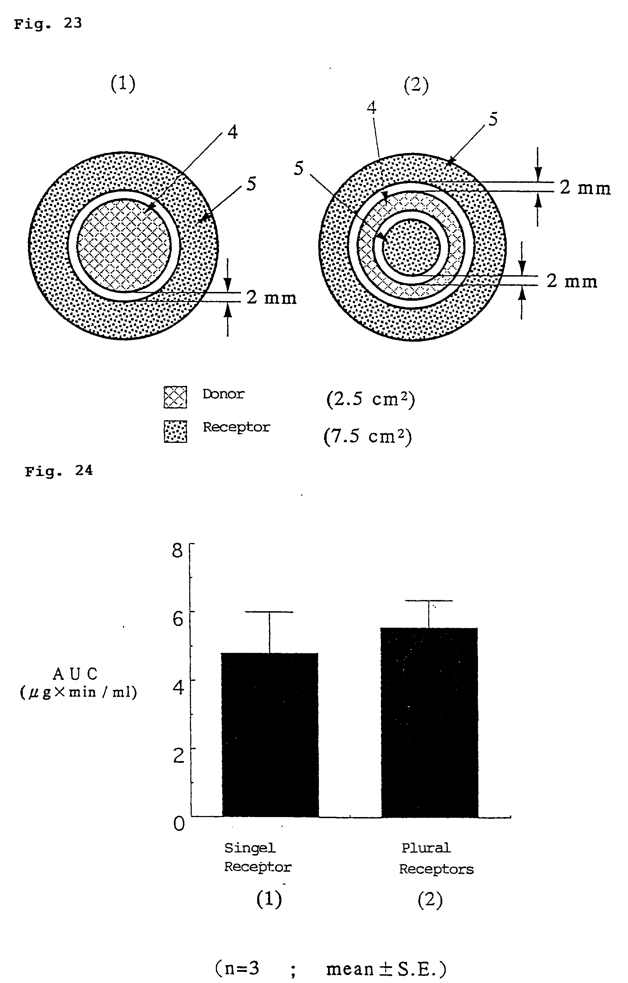

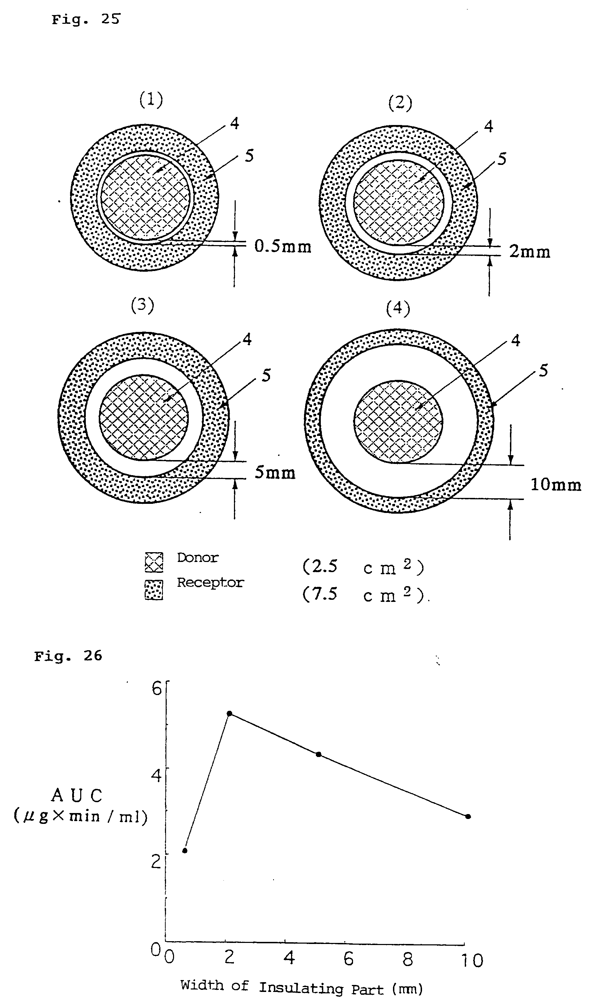

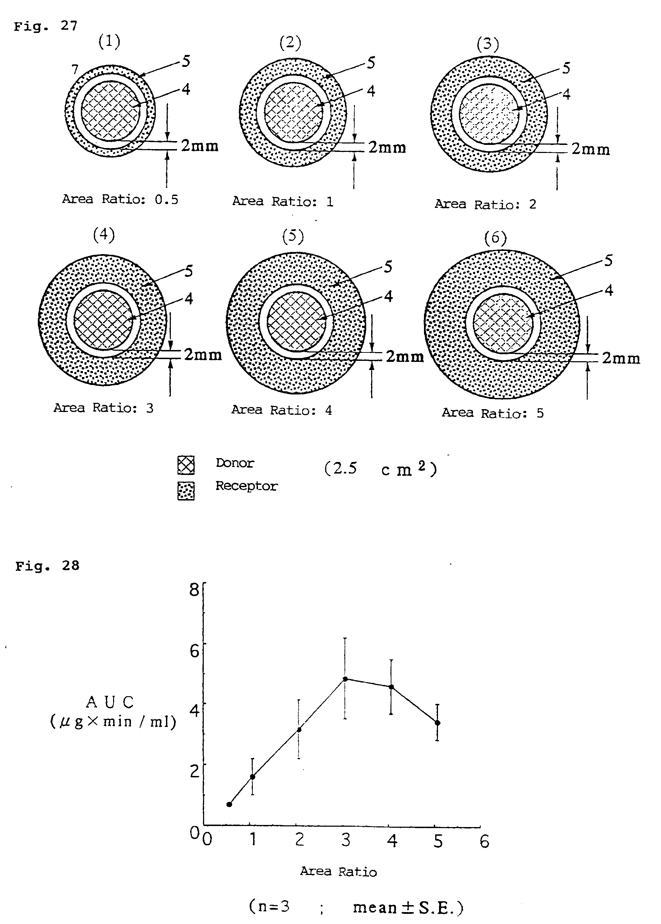

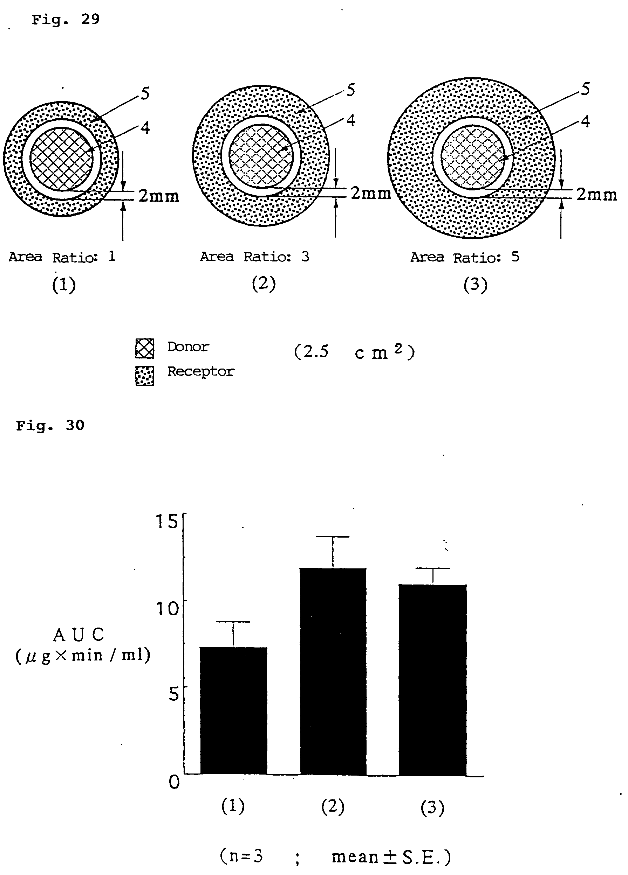

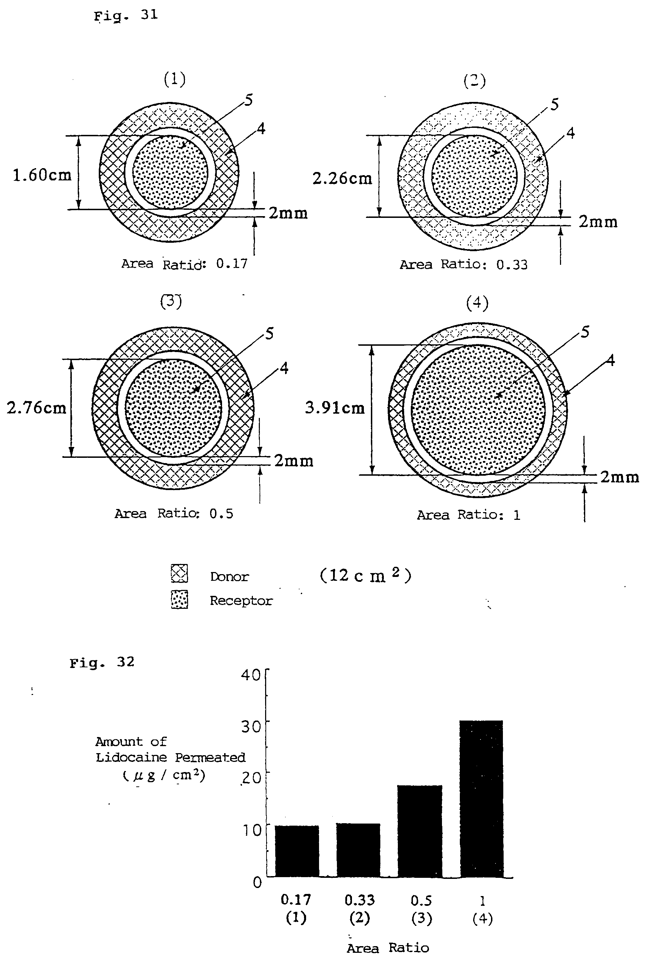

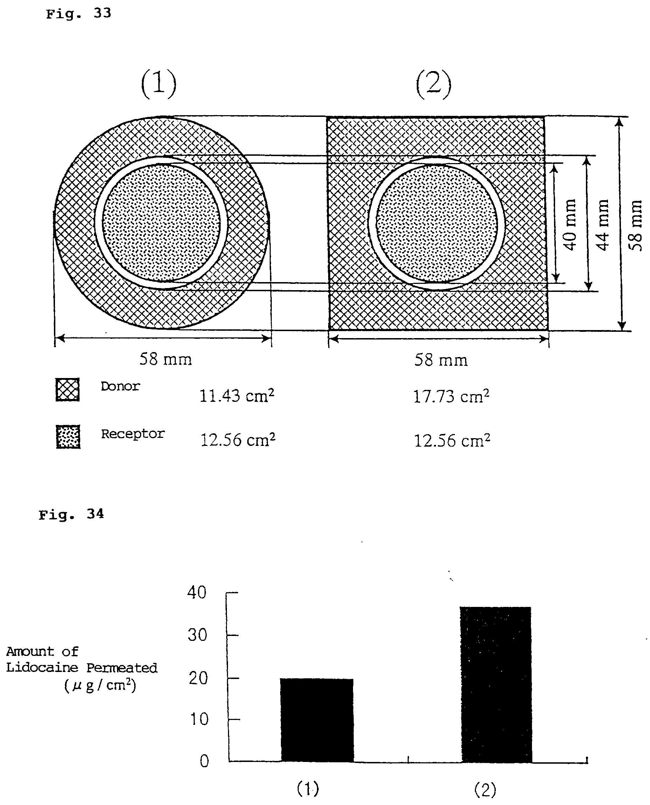

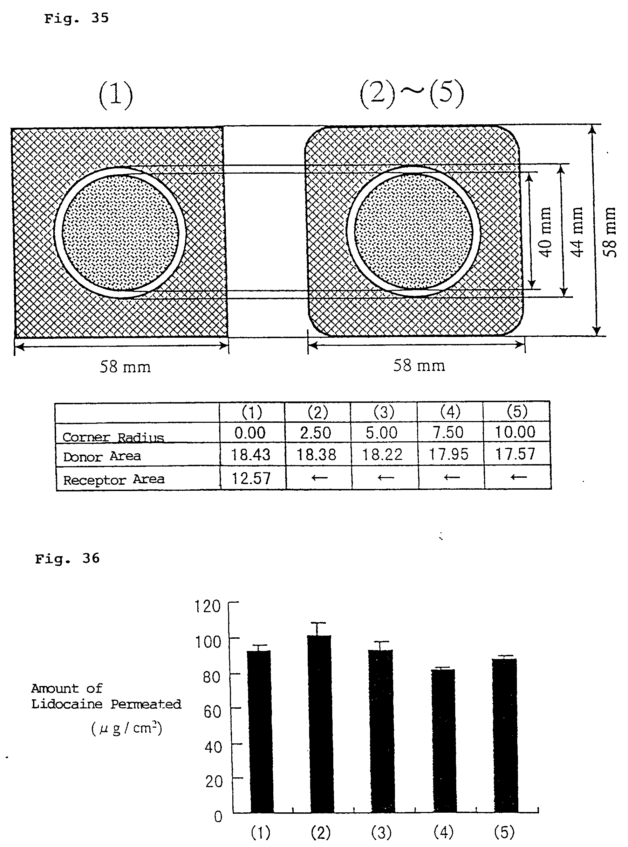

FIG. 1 is a schematic plan view of an iontophoretic device according to the present invention. FIG. 2 is a schematic cross-sectional view of the iontophoretic device according to the present invention. FIG. 3 is a schematic bottom view of the iontophoretic device according to the present invention from which a liner has been peeled off. FIG. 4 is a schematic plan view showing another embodiment of the iontophoretic device according to the present invention. FIG. 5 is a flow chart illustrating production steps for the iontophoretic device according to the present invention. FIG. 6 is a schematic diagram showing the principle of iontophoresis by the iontophoretic device according to the present invention. FIG. 7 is a schematic illustration showing an example of a paired arrangement of a donor and a receptor in the same plane, with the width of an insulating space region varied as will be described in Test 1. FIG. 8 is a graph showing, as a result of Test 1, a relationship between the variations in the width of the insulating space region and AUC. FIG. 9 is a schematic illustration showing an example of a paired and surrounded/surrounding arrangement of a donor and a receptor in Test 2. FIG. 10 is a graph showing, as a result of Test 2, a relationship between the donor-receptor arrangement and AUC. FIG. 11 is a schematic illustration showing examples of a surrounded/surrounding arrangement of a donor and a receptor and examples of the shapes of the donor and receptor. FIG. 12 is a graph showing a relationship between the shapes of the donor and the receptor and AUC as ascertained as a result of Test 3. FIG. 13 is a schematic illustration depicting examples of a surrounded/surrounding arrangement of a donor and a receptor in Test 4. FIG. 14 is a graph showing, as a result of Test 4, a relationship between the surrounded/surrounding arrangement of the donor and the receptor and AUC. FIG. 15 is a schematic illustration showing an example of a paired and surrounded/surrounding arrangement of a donor and a receptor in Test 5. FIG. 16 is a graph showing, as a result of Test 5, a relationship between the donor-receptor arrangement and AUC. FIG. 17 is a schematic illustration showing examples of a surrounded/surrounding arrangement of a donor and a receptor and examples of the shapes of the donor and receptor. FIG. 18 is a graph showing, as a result of Test 6, a relationship between the shapes of the donor and the receptor and AUC. FIG. 19 is a schematic illustration showing examples of a surrounded/surrounding arrangement of a donor and a receptor and examples of the shapes of the donor and receptor. FIG. 20 is a graph showing, as a result of Test 7, a relationship between the kind (space/adhesive tape) of an insulating region between the donor and the receptor and AUC. FIG. 21 is a front illustration of a "PHEO METER" employed in Test 8. FIG. 22 is a graph showing peel strength of an agar pad disclosed in JP Kokai No. 7-185016 and an adhesive pad according to the present invention, which has been obtained as a result of Test 8. FIG. 23 is a schematic illustration showing examples of the number of receptor(s) and a surrounded/surrounding relationship of the receptor(s) with a donor as well as examples of the receptor(s) and donor. FIG. 24 is a graph showing, as a result of Test 9, a relationship between the number of the receptor(s) and AUC. FIG. 25 is a schematic illustration depicting examples of a surrounded/surrounding arrangement of a donor and a receptor in the same plane, with the width of an insulating space region varied as will be described in Test 10. FIG. 26 is a graph showing, as a result of Test 10, a relationship between the variations in the insulating with and AUC. FIG. 27 is a schematic illustration showing examples of a surrounded/surrounding arrangement of a donor and a receptor at different receptor/donor area ratios and examples of the shapes of the donor and receptor, which will be described in Test 11. FIG. 28 is a graph depicting, as a result of Test 11, a relationship between the receptor/donor area ratio and AUC. FIG. 29 is a schematic illustration showing examples of a surrounded/surrounding arrangement of a donor and a receptor at different receptor/donor area ratios and exampies of the shapes of the donor and receptor, which will be described in Test 12. FIG. 30 is a graph depicting, as a result of Test 12, a relationship between the receptor/donor area ratio and AUC. FIG. 31 is a schematic illustration showing examples of a surrounded/surrounding arrangement of a donor and a receptor at different receptor/donor area ratios and examples of the shapes of the donor and receptor, which will be described in Test 13. FIG. 32 is a graph showing, as a result of Test 13, a relationship between the receptor/donor area ratio and an amount of lidocaine permeated in the skin. FIG. 33 is a schematic illustration showing examples of the shape of an outer peripheral portion, one being a circle and the other a square, which will be described in Test 14. FIG. 34 is a graph showing, as a result of Test 14, a comparison in the amount of lidocaine permeated in the skin between the circular outer peripheral portion and the square outer peripheral portion. FIG. 35 is a schematic illustration showing examples of the shape of an outer peripheral portion, one being a square and the other a round-cornered square, which will be described in Test 15. FIG. 36 is a graph showing, as a result of Test 15, a comparison in the amount of lidocaine permeated in the skin between the square outer peripheral portion and the round-cornered square outer peripheral portion.

The following is an explanatory list of symbols in the drawings.
1: power source, 1a: switch, 1b: current application stop control circuit, 2: backing, 3: power source mount, 4: donor, 4A: donor electrode plate, 4B: donor adhesive pad, 5: receptor, 5A: receptor electrode plate, 5B: receptor adhesive pad, 6: insulating space region, 7: liner.

### Best Mode for Carrying Out the Invention

The embodiment of the present invention will hereinafter be described based on FIGS. 1 through 4.

Numeral 1 indicates a power source, which is to be arranged on an upper side of a backing 2. Although this power source 1 may be fixedly assembled beforehand on the upper side of the backing 2, an arrangement of a battery mount 3 on the upper side of the backing 2 to permit detachable mounting of the power source 1 there is more preferred from the standpoint of safety because the power source 1 can be mounted only when the device is used. No particular limitation is imposed on the specific mounting position of the power source 1 insofar as it is on the upper side of the backing 2. The mounting position can therefore be at a central part close to a right side as shown in FIG. 1 or at a lower right corner part as depicted in FIG. 4. Further, it is preferred to additionally provide the backing 2 with an on/off switch 1a for the power source 1 or a circuit 1b for stopping current application in a predetermined time.

Illustrative of the power source 1 for use in the present invention can be a button battery, a cylindrical battery, and a film battery. Use of a film battery is particularly preferred, as it is thin, flexible and free of harmful substances such as mercury, cadmium and lead.

Preferred examples of the backing 2 for use in the present invention can include sheets of plastics such as polyethylene, polypropylene, ethylene-vinyl acetate copolymer, vinylon, polyesters, polyurethanes and nylon; non-woven fabrics of rayon and polyesters; and woven fabrics of polyesters, acrylic resins, silk and cotton. Among non-woven fabrics, a spun lace is particularly advantageous for its high flexibility.

Designated at numerals 4 and 5 are a donor and a receptor, respectively. They are arranged on the same lower side of the backing 2 with an insulating space region 6 interposed therebetween, and are in the form of an integral structure as a whole. Setting of the width of the insulating space region 6, that is, the distance from the donor 4 to the receptor 5 at 0.5 to 12 mm, especially at 1 to 6 mm brings about especially good results in the percutaneous absorption of a drug. Incidentally, if the width of the insulating space region 6 is narrower than the above range, a current passes primarily through a high-resistance stratum corneum in a skin surface, thereby resulting in a reduction in the percutaneous absorption of the drug. On the other hand, a width greater than the above range leads to a reduced current intensity and hence to lowered percutaneous absorption of the drug.

Although no limitation is imposed on the positional relationship between the donor 4 and the receptor 5, it is especially preferred for the attainment of excellent percutaneous absorption efficiency of a drug to arrange the donor 4 and receptor 5 in a surrounding/surrounded or surrounded/surrounding relationship with the insulating space region 6 interposed therebetween as illustrated in FIG. 3.

As the donor 4 for use in the present invention, one having an adhesive pad 4B, which contains a drug solution or a drug and an electrolyte solution, on a lower side of an electrode plate 4A is advantageous from the standpoint of convenience for use in a state attached to the skin. As the receptor 5, on the other hand, one having an adhesive pad 5B, which contains a drug and an electrolyte solution or an electrolyte solution, on a lower side of an electrode plate 5A is advantageous from the same standpoint.

Preferred examples of the electrode plates 4A,5A include those made of one or two metals selected from aluminum (including aluminum oxide), stainless steel, gold, silver, silver chloride, platinum and platinum black.

The drug-containing solution is composed of the drug and a solvent. No particular limitation is imposed on the drug insofar as it can be absorbed percutaneously. Illustrative are protein or peptide preparations; antipyretic, antiphlogistic and analgesic agents; steroidal antiphlogistics; vasodilators; antihypertensive and antiarrhythmic agents; antihypertensive agents; antitussive expectorants; antitumor agents; local anesthetics; hormonal agents; antiallergic agents; antihistamic agents; anticoagulants; antispasmodics; celebral circulation and metabolism improvers; antidepressant anxiolytics; vitamin D preparations; hypoglycemics; antiulcerative agents; hypnotics; antibiotics; antifungal agents; sedatives; bronchodilators; virucides; dysuria treating agents; and parasympathomimetics.

Illustrative of the protein or peptide preparations are insulin, calcitonin, elcatonin, vasopressin, arginine-vasopressin, batroxobin, gonadorelin acetate, octreotide acetate, desmopressin acetate, nafarelin acetate, buserelin acetate, leuprorelin acetate, salmon calcitonin, somatropin, hyaluronidase, prolethilerin, and angiotensin II.

Illustrative of the antipyretic, antiphlogistic and analgesic agents are indomethacin, salicylic acid, sodium salicylate, aspirin, acetaminophen, diclofenac sodium, ibuprofen, sulindac, naproxen, ketoprofen, flufenamic acid, ibufenac, fenbufen, alclofenac, phenylbutazone, mefenamic acid, bendazac, piroxicam, flurbiprofen, pentazocine, buprenorphine hydrochloride, and butorphanol tartrate.

Illustrative of the steroidal antiphlogistics are hydrocortisone, prednisolone, fluocinolone acetonide, fludroxycortide, methylprednisolone, hydrocortisone acetate, triamcinolone acetonide, dexamethazone, betamethasone acetate, diflucortolone valerate, clobetasol propionate, and fluocinonide.

Illustrative of the vasodilators are diltiazem, verapamil, pentaerythritol tetranitrate, dipyridamole, isosorbide nitrate, nifedipine, niconinic acid, and norepinephrine.

Illustrative of the antihypertensive and antiarrhythmic agents are propranolol, atenolol, pindolol, quinidine sulfate, azimaline, alprenolol hydrochloride, metoprolol tartrate, nadolol, timolol maleate, disopyramids, diltiazem hydrochloride, and mexiletine hydrochloride.

Illustrative of the antihypertensive agents are clonidine hydrochloride, captopril, prazosin hydrochloride, penbutolol sulfate, guanabenz acetate, guanfacine hydrochloride, bunazosin hydrochloride, enalapril maleate, arotinolol hydrochloride, binitrolol hydrochloride, and quanethidine sulfate.

Illustrative of the antitussive expectorants are procaterol hydrochloride, terbutaline sulfate, fenoterol hydrobrimide, tulobuterol hydrochloride, ambroxol hydrochloride, pirbuterol hydrochloride, mabuterol hydrochloride, clenbuterol hydrochloride, trimetoquinol hydrochloride, and formoterol fumarate.

Illustrative of the antitumor agents are vincristine sulfate and vinblastine sulfate.

Illustrative of the local anesthetics are benzocaine (hydrochloride), prilocaine (hydrochloride), lidocaine (hydrochloride), tetracaine (hydrochloride), bupivacaine (hydrochloride), and mepivacaine (hydrochloride).

Illustrative of the hormonal agents are steroidal hormones such as estrogen, estradiol, tetrasterone, progesterone, prostaglandin, dinoprost and ritodrine hydrochloride; adrenal hormones such as hydroxycortisone succinate sodium, methylpredonisolone succinate sodium, cortisone acetate, triamcinolone acetate, dexamethasone, hydrocortisone, predonisolone, methylpredonisolone, dexamethasone phosphate sodium hydrocortisone phosphate sodium and epinephrinel; and peptide hormones such as insulin.

Illustrative of the antiallergic agents are ketotifen fumarate, emedastine fumarate, azelastine hydrochloride, sodium cromoglycate, tranilast, cyclosporin, auranofin, tacrolimus hydrate, diphenhydramine hydrochloride, diphenylpyraline hydrochloride, promethazine hydrochloride, diphenylpyraline teoclate, chlorpheniramine maleate, and bucillamine.

Illustrative of the antihistaminic agents are cycloheptadine hydrochloride, diphenhydramine hydrochloride, fenbenzamine, mequitazine and chlorpheniramine maleate.

Illustrative of the anticoagulants are heparin, urokinase, and t-PA.

Illustrative of the antispasmodics are scopolamine, clofloperol, N-methylscopolamine methylsulfate, and papaverine hydrochloride.

Illustrative of the cerebral circulation and metabolism improvers are vinpocetine, flunarizine hydrochloride, nicardipine hydrochloride, brovincamine fumarate, dihydroergotoxine mesylate, ifenprodil tartrate, isoxsuprine hydrochloride, diltiazem hydrochloride, etidronate disodium, and dilazep hydrochloride.

Illustrative of the antidepressant anxiolytics are maprotiline hydrochloride, etizolam, diazepam, bromazepam, amitriptyline hydrochloride, mianserin hydrochloride, chlorpromazine, spiperone, and imipramine hydrochloride.

Illustrative of the vitamin D preparations are alfacalcidol and ergocalciferol.

Illustrative of the hypoglycemics are glibenclamide and glyclazide.

Illustrative of the antiulcerative agents are clebopride malate, famotidine, glycopyrronium bromide, and fluorouracil.

Illustrative of the hypnotics are phenobarbital and amobarbital.

Illustrative of the antibiotics are tetracycline, chloramphenicol, phenoxymethylpenicillin potassium, and erythromycin.

Illustrative of the antifungal agents are ciclopiroxolamine and amphotericin B.

Illustrative of the sedatives are scopolamine hydrobromide, morphine hydrochloride, and fentanyl citrate.

Illustrative of the bronchodilators are theophylline, formoterol fumarate, salbutamol sulfate, terbutaline sulfate, and ephedrine hydrochloride.

Illustrative of the virucides are vidarabine and idoxuridine.

Illustrative of the dysuria treating agents are oxybutynin hydrochloride, arginine-vasopressin, desmopressin acetate, and furosemide.

Illustrative of the parasympathomimetics is acetylcholine chloride.

The proportion of each drug may desirably range from 0.1 to 10 wt.%, although it differs depending on the amount clinically required as its ordinary single dose.

Examples of the solvent can include water; polyhydric alcohols such as ethylene glycol, diethylene glycol, triethylene glycol, ethylene glycol monoethyl ether, ethylene glycol monobutyl ether, diethylene glycol monomethyl ether, polyethylene glycol, propylene glycol, polypropylene glycol, glycerin, batyl alcohol, pentaerythritol, and sorbitol; and alcohols such as ethanol, isopropyl alcohol, lauryl alcohol, cetanol, stearyl alcohol, oleyl alcohol, and lanolin alcohol. They can be used either singly or in combination.

Among these solvents, mixed solvents of polyhydric alcohols, alcohols and water are preferred. Further, polyethylene glycol and propylene glycol are preferred as polyhydric alcohols, and ethanol and isopropyl alcohol are preferred as alcohols. The mixing ratio of these solvents varies depending on the solubility of a drug, but a mixing ratio of polyhydric alcohol:alcohol:water = 0.5-9:0-2:1 is preferred.

In addition, oils and fats, fatty acids, preservatives (antiseptics), stabilizers (antioxidants), surfactants, conductivity-imparting substances and chemical enhancers can be added as needed.

Examples of the oils and fats can include cotton seed oil, olive oil, cacao butter, and palm oil.

Examples of the fatty acids can include propionic acid, butyric acid, valeric acid, caproic acid, heptanoic acid, caprylic acid, nonanoic acid, decanoic acid, undecanoic acid, dodecanoic acid, lauric acid, myristic acid, palmitic acid, palmitoleic acid, stearic acid, oleic acid, linoleic acid, and linolenic acid.

Examples of the preservatives can include phenolic substances such as methyl paraoxybenzoate, phenol and cresol; neutral substances such as chlorobutanol and phenylethyl alcohol; invert soaps such as benzalkonium chloride and benzethonium chloride; and acidic substances such as benzoic acid, sorbic acid, dehydroacetic acid, and salicylic acid.

Examples of the stabilizers can include antioxidants such as vitamin E and butylhydroxyanisole; reducing agents such as ascorbic acid, sodium hydrogen-sulfite and sodium thiosulfate; and synergists such as citric acid (sodium citrate), tartaric acid (sodium tartrate), lecithin, and EDTA.

Examples of the surfactants can include anionic surfactants such as calcium stearate, magnesium stearate and sodium lauryl sulfate; cationic surfactants such as benzalkonium chloride, benzethonium chloride and cetylpyridinium chloride; and nonionic surfactants such as glyceryl monostearate, sucrose fatty acid ester, polyoxyethylene-hydrogenated castor oil, and polyoxyethylene sorbitan fatty acid ester.

Examples of the conductivity-imparting substances can include electrolytes such as sodium chloride, potassium chloride, sodium bromide, potassium bromide, calcium chloride, and calcium bromide.

Examples of the chemical enhancers can include nonionic surfactants such as glyceryl monostearate and sucrose fatty acid ester; water-soluble high molecular compounds such as carboxylic acids; water-soluble chelating agents such as EDTA; aromatic carboxylic acid compounds such as salicylic acid and derivatives thereof; aliphatic carboxylic acid compounds such as capric acid and oleic acid; bile acid salts; propylene glycol; hydrogenated lanolin; isopropyl myristate; diethyl sebacate; urea; lactic acid; and Azone.

As an adhesive polymer for making up adhesive pads in the present invention, a water-soluble polymer or a water-soluble polymer having a crosslinked gel structure is preferred when a hydrophilic drug and a solution are used. Illustrative are polyglucosyloxyalkyl (meth)acrylates, polyhydroxyalkyl (meth)acrylates, polyacrylic acid, polyacrylate salts, polyurethanes, polyvinyl alcohol, polyvinylpyrrolidine, carboxymethylcellulose sodium, gelatin, starch, and agar. Use of a polyglycosyloxyalkyl (meth)acrylate or a polyhydroxyalkyl (meth)acrylate, a polyurethane or polyvinyl alcohol is particularly preferred.

When a hydrophobic drug and a solvent are used, a hydrophobic polymer or a hydrophobic polymer having a crosslinked gel structure is preferred with a polymer of an alkyl (meth)acrylate being especially preferred. Illustrative of the alkyl (meth)acrylate are methyl (meth)acrylate, ethyl (meth)acrylate, n-butyl (meth)acrylate, isobutyl (meth)acrylate, hexyl (meth)acrylate, octyl (meth)acrylate, 2-ethylhexyl (meth)acrylate, isooctyl (meth)acrylate, decyl (meth)acrylate, isodecyl (meth)acrylate, lauryl (meth)acrylate, and stearyl (meth)acrylate. In particular, use of n-butyl acrylate or 2-ethylhexyl acrylate, which has been widely used as an adhesive component for cataplasma, is preferred.

Hydrophilic monomers and hydrophobic monomers can be used either singly or in combination. If necessary, a hydrophilic monomer and a hydrophobic monomer can be used in combination

In the present invention, it is particularly advantageous for the achievement of excellent percutaneous absorption effects for a drug to form the donor 4 into a regular polygonal shape which may be rounded at corners, especially one having 6 or more corners or an oval or circular shape and also to form the receptor 5 into a polygonal shape which may be a round-cornered square shape, especially one having 6 or more corners or a oval or circular shape. Incidentally, an arrangement of the donor 4 and the receptor 5 in a surrounded/surrounding or surrounding/surrounded relationship allows a current to primarily flow through corner portions, so that the distribution of current intensity becomes more even as the number of regular corners in the shape of the donor 4 or receptor 5 increases.

In the present invention, it is especially preferred for the achievement of excellent percutaneous absorption effects of a drug to arrange a donor and a receptor in a surrounded/surrounding or surrounding/surrounded relationship and also to make the area a skin-contacting portion of the donor or receptor, which is located at the outer peripheral area, 1 to 6 times as large as the area of a skin-contacting portion of the receptor or donor located at the central area.

In the present invention, it is preferred from the standpoint of storage and management during non-use to peelably cover the surfaces of the adhesive pads 4B,5B with a liner 7. Preferred examples of the liner 7 can be plastic films and a cellulose film.

The iontophoretic device of this invention constructed as described above can be produced through steps similar to those employed for general medical adhesives such as adhesive plasters and antiphlogistic and analgesic plasters as shown by way of example in the production step flow chart of FIG. 5. A description will next be made about principal steps.

### 1) Preparation of adhesive base

Ethylene glycol dimethacrylate (crosslinking agent) and azobisisobutyronitrile (polymerization initiator) are added to 2-hydroxyethyl methacrylate and n-butyl acrylate, and the resultant mixture is subjected to solution polymerization while using dimethylformamide as a polymerization solvent. After the polymerization, an adhesive base is produced through filtration → washing and purification → filtration → drying → grinding → packaging steps.

### 2) Preparation of drug solution

In a mixed solvent of a polyhydric alcohol, an alcohol and water, a drug is dissolved only in an amount clinically required as a single dose. At this time, an oil or fat, a fatty acid, a preservative (antiseptic), a stabilizer (antioxidant), a surfactant, a conductivity-imparting substance and a chemical absorption enhancer can be added as needed.

### 3) Mixing step

The adhesive base and the drug solution are kneaded at a predetermined mixing ratio.

### 4) Spreading step

A mixture, which has been formed in the mixing step, is evenly spread thin on an electrode. Here, the electrode is selected from aluminum (oxide), stainless steel, gold, silver, silver chloride, platinum or platinum black. The thus-spread thickness may be from 0.1 to 1 mm, with 0.2 to 1 mm being particularly preferred.

### 5) Cutting step

The electrode with the mixture evenly spread thin thereon is cut by a cutter formed in a desired shape so that at the same time, the electrode and the mixture in an insulating region portion are removed.

### 6) Backing applying step

A protective film is applied on a surface of the plaster which has been obtained through the cutting step. An adhesive is coated on an electrode surface, on which a backing cut in the desired shape is adhered. Here, the backing is selected from a plastic sheet, a non-woven fabric or a woven fabric, and a spun lace as a non-woven fabric is particularly preferred. No particular limitation is imposed on the adhesive insofar as it is generally used for medical purposes. However, an acrylic adhesive is preferred.

### 7) Insulating material coating step

Portions other than the electrode and the skin-contacting surface of the plaster are coated with an insulating material. No particular limitation is imposed on the insulating material insofar as it is generally used for medical purposes. However, polymers such as polyvinyl chloride, polyesters and butyl rubber are preferred.

### 8) protective film applying step

A protective film is applied to a skin-contacting surface of an assembly which has been obtained by mounting the backing on the plaster and coating the portions other than the electrode and the skin-contacting surface of the plaster with the insulating material.

### 9) Manufacture of power source

A power source is manufactured through a positive and negative electrode plate forming step, a polymer electrolyte film forming step, an assembly step, a terminal connecting step, an insulating material coating step, an inspection and packaging. In this manufacture process, additional steps may also be incorporated to introduce functions such as switching, frequency control, time control and the like.

The liner 7 is peeled off from the iontophoretic device of this invention obtained as described above. After the adhesive pads 4B,5B of the donor 4 and receptor 5 are applied to the skin, a current is applied. As is illustrated in the schematic diagram of FIG. 6, a flow of electrons from a cathode (donor) to an anode (receptor) takes place as shown in the schematic diagram of FIG. 6 when indomethacin as an anionic drug is used, for example. By this flow, anions in the cathode are caused to migrate to the anode and on the other hand, cations in the anode are caused to migrate to the cathode, whereby the drug component is percutaneously absorbed.

### Examples

The present invention will hereinafter be described further by the following Examples and Tests.

### Example 1

Using the below-described power source, backing, donor, receptor and liner, an iontophoretic device according to the present invention as shown in FIGS. 1 through 3 was produced in accordance with the production steps illustrated in FIG. 5.

### 1) Power source

A single film battery (voltage: 1.5 V or 3 V) was used.

### 2) Backing

A non-woven fabric (spun lace: 80 cm²) was used.

### 3) Donor and receptor

Those produced as described below were employed.

### (i) Synthesis of adhesive polymer:

Mixed were 100 g of a blend of 2-hydroxymethyl methacrylate (hereinafter referred to as "HEMA"), n-butyl acrylate (hereinafter referred to as "BA") and ethylene glycol dimethacrylate (hereinafter referred to as "EGDMA") at HEMA:BA:EGDMA = 2:1:0.125 (molar ratio), 0.5 g of 2,2'-azobisisobutyronitrile and 100 g of dimethylformamide. Subsequent to purging with nitrogen gas for deaeration, a polymerization reaction was conducted at 75°C for 15 hours. After stopping of the polymerization reaction, procedures (swelling in isopropyl alcohol → removal of isopropyl alcohol by decantation → washing with deionized water → vacuum drying → grinding) were repeated twice to conduct purification. The thus-purified polymerization product was passed through an 83-mesh sieve, whereby a fine pulverized polymer was obtained.

### (ii) Preparation of indomethacin solution:

Using the formula described in Table 1, indomethacin was dissolved in isopropyl alcohol, followed by the addition of polyethylene glycol 400 and saline to prepare an indomethacin solution.

**Table 1**

| Formula of indomethacin solution | |
|---|---|
| Indomethacin | 1.2 g |
| Isopropyl alcohol | 24 g |
| Polyethylene glycol 400 | 49 g |
| Saline | Added to 100 g |

### (iii) Production of indomethacin-containing adhesive pad:

With 1 part by weight of the polymer obtained in step (i), 5 parts of the indomethacin solution obtained in step (ii) were mixed. The thus-prepared coating formulation was spread into a mold with 0.5 mm thickness in an amount of 0.1 g per cm² between silicone sheets, whereby an indomethacin-containing adhesive pad was produced.

### (iv) Electrodes:

A disc-shaped electrode made of stainless steel was used as a donor electrode, while a doughnut-shaped electrode made of stainless steel was employed as a receptor electrode.

### Test 1 - Comparison of indomethacin absorption when the distance from a donor to a receptor (the width of an insulating space region) was changed in the same plane:

After hair was removed from abdominal regions of Wistar rats (male, 8 weeks old), rectangular donor and receptor adhesive pads produced as in Example 1 were adhered in pairs side by side at varied distances therebetween as illustrated in FIG. 7. By a constant voltage device, a voltage was applied at 4.5 V for 60 minutes. Upon elapsed time of 20, 40 and 60 minutes after starting the voltage application, 1 mℓ blood samples were collected and their concentrations of indomethacin in blood plasma were analyzed by HPLC. Changes in AUC (area under the plasma time change course curve) by the width of the insulating space region are shown in FIG. 8. The AUC tended to increase up to a donor-receptor distance of 2 mm but to decrease at donor-receptor distances of 4 mm and greater.

### Test 2 - Comparison of indomethacin absorption between a paired arrangement of a donor and a receptor and a surrounding arrangement of a receptor at an outer peripheral area of a donor, each in the same plane with an insulating space region of 2 mm in width interposed therebetween:

In a similar manner as in Test 1 except that the shapes and relative positions of donor and receptor having adhesive pads produced as in Example 1 were changed as shown in FIGS. 9(1) and 9(2), a voltage was applied at 3 V for 60 minutes, and the concentrations of indomethacin in blood plasma were analyzed. A comparison of the AUC is shown in FIG. 10. Although the donor and receptor areas and the donor-receptor distance were the same, the surrounding arrangement of the receptor at an outer peripheral area of the donor was greater in AUC.

### Test 3 - Comparison of indomethacin absorption among different donor and receptor shapes in the case of surrounding arrangements of receptors at outer peripheral areas of their corresponding donors with insulating space regions of 2 mm in width interposed therebetween:

In a similar manner as in Test 1 except that the shapes and relative positions of donor and receptor having adhesive pads produced as in Example 1 were changed as shown in FIGS. 11(1) to 11(4), a current was applied at 0.16 mA for 60 minutes, and the concentration of indomethacin in blood plasma were analyzed. A comparison of the AUC is shown in FIG. 12. The AUC increased as the number of corners increased in the order of a triangle, a square and a hexagon. No substantial difference was however observed between the hexagon and a circle.

### Test 4 - Comparison in indomethacin absorption between a surrounding arrangement of a receptor at an outer peripheral area of a donor and a surrounding arrangement of a donor of the same area as the first-mentioned donor at an outer peripheral area of a receptor of the same area as the first-mentioned receptor, each in the same plane with an insulating space region of 2 mm in width interposed therebetween:

In a similar manner as in Test 1 except that the shapes and relative positions of donor and receptor were changed as shown in FIGS. 13(1) and 13(2), a voltage was applied at 3 V for 60 minutes, and the concentrations of indomethacin in blood plasma were analyzed. A comparison of the AUC is shown in FIG. 14. The AUC was substantially the same.

### Example 2

An iontophoretic device according to the present invention was produced in a similar manner as in Example 1 except that a lidocaine solution of the formula shown in Table 2 was employed as donor and receptor solutions.

**Table 2**

| Formula of lidocaine solution | |
|---|---|
| Lidocaine | 5 g |
| Polyethylene glycol 400 | 80 g |
| Saline | Added to 100 g |

### Test 5 - Comparison in lidocaine absorption between a paired arrangement of a donor and a receptor and a surrounding arrangement of a receptor at an outer peripheral area of a donor, each in the same plane with an insulating space region of 2 mm in width interposed therebetween:

After hair was removed from abdominal regions of Wistar rats (male, 8 weeks old), lidocaine-containing, donor and receptor adhesive pads produced as in Example 2 were adhered in such shapes and arrangements as illustrated in FIGS. 15(1) and 15(2). By a constant voltage device, a voltage was applied at 3 V for 180 minutes. Upon elapsed time of 60, 120 and 180 minutes after starting the voltage application, 1 mℓ blood samples were collected and their concentrations of lidocaine in blood plasma were analyzed by HPLC. A comparison of the AUC is shown in FIG. 16. The surrounding arrangement of the receptor at the outer peripheral area of the donor was clearly higher in AUC.

### Test 6 - Comparison in indomethacin absorption between a polygon and a regular polygon:

After hair was removed from abdominal regions of Wistar rats (male, 8 weeks old), donors and receptors - which were composed of stainless steel electrodes and adhesive pads (produced as in Example 1) and were in the shapes and arrangements shown in FIGS. 17(1) and 17(2) - were adhered with insulating space regions of 2 mm in width interposed therebetween. They were fixed by surgical tapes, and each cathode (donor), its corresponding anode (receptor) and a constant voltage device were connected together by lead wires. A voltage was applied at 4.5 V for 60 minutes. Upon elapsed time of 20, 40 and 60 minutes after starting the voltage application, 1 mℓ blood samples were collected and their concentrations of indomethacin in blood plasma were analyzed by HPLC. A comparison in AUC between the polygon (octagon) and the regular polygon (regular octagon) is shown in FIG. 18. Although the donor and receptor areas and the insulating regions were of the same length, the regular polygon was clearly higher in AUC.

### Test 7 - Comparison in indomethacin absorption between a space and an adhesive tape as an insulating region of 2 mm in width in the case of a regular polygon:

When the insulating region was the space, the concentrations of indomethacin were analyzed in a similar manner as in Test 6 except that donors and receptors - which were composed of stainless steel electrodes and pads and were in the shapes and arrangements shown in FIGS. 19(1) and 19(4) - were used and the voltage application conditions were set at 3 V - 60 minutes. When the insulating region was the adhesive tape, on the other hand, the concentrations of indomethacin in blood plasma were analyzed in a similar manner as in the case of the space as the insulating region except that a silicone rubber with 0.5 mm thickness was placed at the insulating region and a double-tack tapes ("NICE TACK", trade name; product of Nichiban Co., Ltd.) were used at contacting areas to the skin. A comparison in AUC between the case of the space as the insulating region and the case of the adhesive tape as the insulating region is shown in FIG. 20. In the case of the space as the insulating region, the AUC increased with the number of corners in the polygon, achieving the highest AUC in the case of the circle. According to the comparison between the space an the adhesive tape as the insulating area, the space was higher in AUC in any one of the regular polygons. This difference tended to become greater as the number of corners increased.

### Test 8 - Adhesion tests of pads:

The indomethacin-containing adhesive pad (2 cm x 2 cm x 0.5 cm thick) produced in Example 1 and an indomethacin-containing agar pad (2 cm x 2 cm x 0.5 cm) disclosed in JP Kokai No. 7-185016 were each placed on a slide glass which was mounted on a movable table of "RHEO METER" (Model: NRM-3002D-L; manufactured by Fudo Kogyo K.K.) shown in FIG. 21. After a cover glass (1.8 cm x 1.8 cm; area: 3.24 cm²) attached to an adaptor was pressed until a load of 250 g was applied on the pad, the instrument was left over for 10 seconds and the movable table was moved at a speed of 2 cm/min to peel off the pad. A load at the time of peeling-off was measured. A comparison in peel strength between the agar pad and the indomethacin-containing pad produced in Example 1 is shown in FIG. 22. The indomethacin-containing pad was clearly higher in peel strength and, when adhered on a human forearm, did not separate. In contrast, the agar pad was low in peel strength and, when adhered on a forearm, separated shortly.

### Test 9 - Comparison in indomethacin absorption between a single receptor and plural receptors:

Plasma indomethacin levels were analyzed in a similar manner as in Test 6 except that the voltage application conditions were set at 3 V - 60 minutes and the donors and receptors - which were composed of electrodes and adhesive pads and were in the shapes and arrangements shown in FIGS. 23(1) and 23(4) - were used with insulating space regions of 2 mm in width interposed therebetween. A comparison in AUC between the single receptor and the plural receptors is shown in FIG. 24. The AUC was substantially equal irrespective of the number of receptor(s).

### Test 10 - Comparison of indomethacin absorption among different distances from a donor to a receptor in a concentric arrangement:

The concentrations of indomethacin in blood plasma were analyzed in a similar manner as in Test 6 except that the voltage application conditions were set at 3 V - 60 minutes and donors and receptors - which were composed of electrodes and adhesive pads and were in the shapes and arrangements shown in FIGS. 25(1) and 25(4) - were used with insulating space regions of 0.5 mm, 2 mm, 5 mm and 10 mm in width interposed therebetween. A comparison of AUC among the insulating regions of the different widths in the concentric arrangement is shown in Table 26. The AUC reached the maximum when the width of the insulating space region was 2 mm.

### Test 11 - Comparison of indomethacin absorption among different receptor/donor area ratios in a concentric arrangement:

Plasma indomethacin levels were analyzed in a similar manner as in Test 6 except that the voltage application conditions were set at 3 V - 60 minutes and donors and receptors - which were composed of electrodes and adhesive pads, were in the shapes and arrangements shown in FIGS. 27(1) through 27(6), and had receptor/donor area ratios of 0.5, 1, 2, 3, 4 and 5 - were used with insulating space regions of 2 mm in width interposed therebetween. A comparison of the AUC among the different receptor/donor area ratios in the concentric arrangement is shown in FIG. 28. The AUC apparently increased up to the area ratio of 3, but no substantial changes were observed beyond that area ratio.

### Test 12 - Comparison of lidocaine absorption among different area ratios of a receptor (outer peripheral part) to a donor (inner central part):

The concentrations of lidocaine in blood plasma were analyzed in a similar manner as in Test 5 except that donors and receptors - which were composed of electrodes of the shapes as shown in FIGS. 29(1) to 29(3) and lidocaine-containing donor and receptor adhesive pads produced as in Example 2 and had receptor/donor area ratios of 1, 3 and 5 - were used and arranged as shown in the same drawings. A comparison of the AUC among the different receptor/donor area ratios in the concentric arrangement is shown in FIG. 30. The AUC increased up to the area ratio of 3, but no substantial changes were observed beyond that area ratio.

### Test 13 - Comparison of lidocaine absorption among different area ratios of a receptor (inner central part) to a donor (outer peripheral part) in a concentric arrangement:

Employed were donors and receptors, which were composed of electrodes of the shapes shown in FIGS. 31(1) to 31(4) and lidocaine-containing donor and receptor adhesive pads produced as in Example 2 and had receptor/donor area ratios of 0.17, 0.33, 0.5 and 1. The donors and receptors were arranged as shown in the same drawings, and stainless steel electrodes of the same shapes as the pads were attached. A voltage was applied at 3 V for 40 minutes by a constant voltage device.

After stopping the voltage application, the pads were peeled off and skins were then wiped with 70% alcohol. Skin samples were excised from the pad-adhered parts, and the amounts of lidocaine permeated into the skins were analyzed by HPLC. Comparison results are shown in FIG. 32.

The amount of lidocaine permeated increased with the receptor/donor area ratio when the area of the donor and the width of the insulating region were the same.

### Test 14 - Comparison in lidocaine absorption between a receptor (inner central part) and a donor (outer peripheral part) having the same shape and those having different shapes:

Electrodes of the shapes shown in FIGS. 33(1) to 33(2) and lidocaine-containing donor and receptor adhesive pads produced as in Example 2 were adhered to hair-removed abdominal regions of rats. Stainless steel electrodes of the same shapes as the corresponding pads were attached. A voltage was applied at 3 V for 20 minutes by a constant voltage device.

After stopping the voltage application, the amount of lidocaine permeated in the skin was analyzed by HPLC in a similar manner as in Test 13. Their comparison is shown in FIG. 34.

The amount of lidocaine permeated was greater in the case of the square peripheral part when the areas of the receptor and the insulating region were the same.

### Test 15 - Comparison of lidocaine absorption among a square donor with unrounded corners and square donors with rounded corners in the combinations of circular receptors (inner central parts) and the square donors (outer peripheral part):

Electrodes of the shapes shown in FIGS. 35(1) to 35(5) and lidocaine-containing donor and receptor adhesive pads produced as in Example 2 were adhered to hair-removed dorsal regions of guinea pigs. Stainless steel electrodes of the same shapes as the corresponding pads were attached. A voltage was applied at 3 V for 120 minutes by a constant voltage device.

After stopping the voltage application, the amount of lidocaine permeated in the skin was analyzed by HPLC in a similar manner as in Test 13. Comparison results are shown in FIG. 36.

The amount of lidocaine permeated was substantially equal among the case of the square outer peripheral part and the cases of the square peripheral parts with the corners thereof rounded at radii of up to 10 mm.

### Capability of Exploitation in Industry

The present invention can provide am iontophoretic device, which permits safe and efficient percutaneous absorption of a drug without increasing a current value and also features extremely good handling ease.

## Claims

1. An iontophoretic device for the electric percutaneous administration of a drug, comprising: a backing provided with a power source, and a donor and a receptor arranged on the same one side of said backing with an insulating space region interposed therebetween, whereby said device has an integral structure.

2. An iontophoretic device according to claim 1, wherein said receptor is arranged surrounding an outer peripheral part of said donor or said donor is arranged surrounding an outer peripheral part of said receptor, with said insulating space region interposed therebetween.

3. An iontophoretic device according to claim 1 or 2, wherein said insulating space region interposed between said receptor and said donor has a width of from 0.5 to 12 mm.

4. An iontophoretic device according to any one of claims 1-3, wherein said donor is provided on one of sides of an electrode plate with an adhesive pad containing a solution of said drug or said drug and an electrolyte solution, and said receptor is provided on one of sides of an electrode plate with an adhesive pad containing said drug and an electrolyte solution or an electrolyte solution.

5. An iontophoretic device according to any one of claims 1-4, wherein said donor and said receptor are in the form of regular polygonal shapes which may be round-cornered square shapes, oval shapes or circular shapes.

6. An iontophoretic device according to any one of claims 1-5, wherein said donor and said receptor is in a surrounding/surrounded or surrounded/surrounding arrangement relationship, and an area of a skin-contacting portion of said outer peripheral part is 1 to 6 times as large as an area of a skin-contacting portion of said inner central part.

7. An iontophoretic device according to any one of claims 1-6, wherein said drug is a protein or peptide preparation, an antipyretic, antiphlogistic and analgesic agent, a steroidal antiphlogistic, a vasodilator, an antihypertensive and antiarrhythmic agent, an antihypertensive agent, an antitussive expectorant, an antitumor agents, a local anesthetic, a hormonal agent, an antiallergic agents, an antihistamic agent, an anticoagulant, an antispasmodic, a cerebral circulation and metabolism improver, an antidepressant anxiolytic, a vitamin D preparation, a hypoglycemic, an antiulcerative agent, a hypnotic, an antibiotic, an antifungal agent, a sedative, a bronchodilator, a virucide, a dysuria treating agent, or a parasympathomimetics.

8. An iontophoretic device according to any one of claims 1-7, wherein said adhesive pads are made of polyvinyl alcohol, polyurethane, or crosslinked copolymer of at least two esters selected from glucosyloxyalkyl (meth)acrylates, hydroxyalkyl (meth)acrylates or alkyl (meth)acrylates.

9. An iontophoretic device according to any one of claims 1-8, wherein said electrodes are made of one or two metals selected from aluminum, stainless steel, gold, silver, silver chloride, platinum or platinum black.

10. An iontophoretic device according to any one of claims 1-9, wherein said backing comprises any one of polyethylene, polypropylene, ethylene-vinyl acetate copolymer, vinylon, polyester, polyurethane and nylon sheets, non-woven rayon and polyesters fabrics, and woven polyester, acrylic resin, silk and cotton fabrics.

11. An iontophoretic device according to any one of claims 1-10, wherein said power source is a button battery, a cylindrical battery or a film battery.

12. An iontophoretic device according to any one of claims 1-11, wherein said adhesive pads are peelably covered at surfaces thereof with liners.

13. An iontophoretic device according to claim 12, wherein said liners are plastic films or cellulose films.
